# EUROPÄISCHE PATENTSCHRIFT

(11) **EP 0 863 110 B1**
(45) Veröffentlichungstag und Bekanntmachung des Hinweises auf die Patenterteilung: **24.07.2002**
(21) Anmeldenummer: 98103719.5
(22) Anmeldetag: 03.03.1998
(51) Int. Cl.: C01C 3/02, C07C 11/24, C07C 1/32

(54) **Verfahren zur simultanen Herstellung von Acetylen und Blausäure aus Acrylnitril**
Process for the simultaneous preparation of acetylene and hydrogen cyanide from acrylonitrile
Procédé pour la production simultanée d'acétylène et d'acide cyanhydrique à partir d'acrylonitrile

(30) Priorität: 06.03.1997 DE 19709261
(43) Veröffentlichungstag der Anmeldung: 09.09.1998
(73) Patentinhaber: BASF AKTIENGESELLSCHAFT, 67056 Ludwigshafen (DE)
(72) Erfinder: Halbritter, Klaus, Dr., 69124 Heidelberg (DE); Henningsen, Michael, Dr., 67227 Frankenthal (DE); Julius, Manfred, Dr., 67117 Limburgerhof (DE); Stegmaier, Wolf, Dr., 67459 Bölh-Iggelheim (DE)
(74) Vertreter: Isenbruck, Günter, Dr.

(56) Entgegenhaltungen:
- US-A- 4 136 156
- T. MORIKAWA : "Evolution of hydrogen cyanide during combustion and pyrolysis." JOURNAL OF COMBUSTION TOXICOLGY+, Bd. 5, Nr. 3, August 1978, Seiten 31315-330, XP002069700
- E. METCALFE ET AL.: "The pyrolysis of organic nitriles." FIRE AND MATERIALS., Bd. 7, Nr. 4, 1983, Seiten 185-192, XP002069701
- N.P. MACHARA ET AL.: "The 193-nm excimer laser photofragmentation of alkane and alkane nitriles in argon matrices." JOURNAL OF PHYSICAL CHEMISTRY., Bd. 92, Nr. 22, 1988, Seiten 6241-6245, XP002069702
- T.W. ASMUS ET AL.: "Pyrolysis kinetsics of acetonitrile." JOURNAL OF PHYSICAL CHEMISTRY., Bd. 73, Nr. 8, August 1969, Seiten 2555-2558, XP002069703

## Beschreibung

Die Erfindung betrifft ein Verfahren zur simultanen Herstellung von Acetylen und Blausäure.

Acetylen und Blausäure, aus denen eine Vielzahl an organischen Grund- und Zwischenprodukten hergestellt wird, sind wichtige Synthesebausteine in der chemischen Industrie. Ein Herstellungsweg für Acetylen, nämlich die Herstellung durch oxidative Methankupplung, erfordert hochintegrierte Standorte, da nur so die Reaktions-"Nebenprodukte" sinnvoll verwendet werden können. So fallen bei dem genannten Verfahren zur Acetylen-Herstellung (Öl-Quench-Verfahren) pro Tonne Acetylen u.a. 10600 m³ Roh-Synthesegas, 13 t Dampf und 0,3 t Ruß an (Angaben aus Ullmann's Encyclopedia of Industrial Chemistry, 5. Ed. - 1985 , Vol. A1, S. 112).

Verfahren, die alternative selektive Zugänge zu Acetylen unter gleichzeitiger Herstellung von Blausäure bereitstellen, sind deshalb sehr interessant: A. Lifshitz et al. [Int. J. Chem. Kinet. Vol. *19*, 61-79 (1987); Combustion and Flame *78*, 43-57 (1989); J. Phys. Chem. *93*, 1369-1373 (1989)] untersuchten die Pyrolyse von N-haltigen organischen Molekülen unter Anwendung von reflektierten Schockwellen (single-pulse shock tube technique). Unter Anwendung dieser Technik wurden dabei, ausgehend von Acrylnitril (1 % H₂C=CH-CN in Argon), bei einer Temperatur von 1425K und einem Umsatz von ca. 25 % Blausäure und Acetylen mit einer Selektivität von je ca. 48 % - d.h. Ausbeuten von lediglich je ca. 12 % - erhalten. Das single-pulse-shock-Verfahren zur Zersetzung von Acrylnitril ist jedoch eine diskontinuierliche Methode, die für eine ökonomische simultane Produktion von Blausäure und Acetylen völlig ungeeignet ist, da kein kontinuierlich betreibbarer chemischer Reaktor eingesetzt werden kann. Zudem muß zur Erzielung eines Acrylnitril-Umsatzes von 40 % eine Temperatur von mindestens 1450K (1177 °C) angewendet werden.

Aus der US 4,136,156 ist bekannt, daß bei der Thermolyse von organischen Nitrilen, wie Benzonitril, bei 400 bis 700 °C in Gegenwart von Edelmetallkatalysatoren und Wasserstoff Blausäure entsteht.

Weiterhin ist in DE-A 16 68 102 beschrieben, daß man Acetylen durch Enthalogenierung von Dihalogenethan oder Vinylbromid (Vinylchlorid) in Gegenwart basischer Katalysatoren, wie BaO, herstellen kann.

Der Artikel "Evolution of hydrogen cyanide during combustion and pyrolysis" von T. Morikawa in Journal of Combustion Toxicology, Vol. 5 (8/1978), S. 315-330 befasst sich mit Pyrolyse- und Verbrennungsreaktionen von Stickstoffverbindungen, aber ohne Relevanzbezug zur Abkühldauer.

Der Artikel "The pyrolysis of organic nitriles" von E. Metcalfe et al. in Fire and Materials, Vol. 7, No. 4, 1983, S. 185-192 enthält Laborversuche über die Pyrolyse von Nitrilen in einer Pyrolyse-GC-Apparatur ohne die Möglichkeit der Isolation der Reaktionsprodukte.

Aufgabe der Erfindung ist es, ein Verfahren zu entwickeln, bei dem aus Acrylnitril die beiden Produkte Acetylen und Blausäure hergestellt werden. Dieses Verfahren soll insbesondere auch bei der industriellen Anwendung vorteilhaft einsetzbar sein.

Diese Aufgabe wird durch ein Verfahren zur Herstellung von Acetylen und Blausäure gelöst, bei dem Acrylnitril einer Pyrolyse in einem Reaktor unterworfen wird und die bei der Pyrolyse gebildeten gasförmigen Reaktionsprodukte innerhalb von ≤ 1 s nach Verlassen der Pyrolysezone auf Temperaturen kleiner 100°C abgeschreckt werden. Die Form des Reaktors spielt dabei eine eher untergeordnete Rolle; zweckmäßig sind solche Reaktoren, die gegebenenfalls mit einem inerten Material gefüllt sind, in denen eine höhere Temperatur in der Größenordnung von 1000°C mit einer kurzen Verweilzeit des zu pyrolysierenden Gases von 1 sec oder Bruchteilen davon - insbesondere einschließlich einer sich anschließenden Abkühlphase - realisiert werden kann. Vom Grundprinzip her sind solche Reaktoren beispielsweise in Acetylenbrennern verwirklicht.

Bereitgestellt wird dementsprechend erfindungsgemäß ein Verfahren zur simultanen Herstellung von Acetylen und Blausäure, das durch die pyrolytische Dehydrocyanierung von Acrylnitril nach der allgemeinen Gleichung 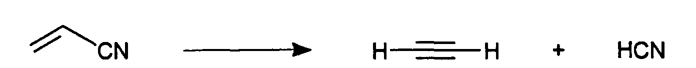 beschrieben wird.

Die erfindungsgemäße Acrylnitril-Pyrolyse kann in Gegenwart eines inerten Gases, wie Stickstoff oder Argon, durchgeführt werden. In einer bevorzugten Ausführungsform des erfindungsgemäßen Verfahrens kann die Umsetzung kontinuierlich in einem Pyrolysereaktor erfolgen, der eine Reaktionszone aus z.B. keramischem Material aufweist. Ein Pyrolysereaktor, der für das erfindungsgemäße Verfahren geeignet ist, ist beispielhaft in der US-A 4,973,777 beschrieben. Die Pyrolyse des Acrylnitrils, gegebenenfalls unter Zusatz eines Inertgases, ist vorteilhaft bei Temperaturen zwischen 900 und 1100 °C und Verweilzeiten kleiner 1 s. Bevorzugt sind Verweilzeiten von ≤ 0,2 s, insbesondere ≤ 0,1 s. Die Ausbeute an Acetylen beträgt bei einer Verweilzeit von 0,1 sec 34,2 % und die Ausbeute an Blausäure 53,4 %. Das erfindungsgemäße Verfahren kann drucklos oder unter Druck, in beiden Fällen vorteilhafterweise jedoch unkatalysiert durchgeführt werden.

Überraschenderweise gelingt die Acrylnitril-Pyrolyse nach vorstehender Gleichung unter Anwendung des erfindungsgemäßen Verfahrens ― wie vorstehend erwähnt ― bereits bei einer Temperatur von etwa 1000 °C mit einem Acrylnitril-Umsatz von > 60 %.

Das erfindungsgemäße Verfahren wird vorteilhafterweise in einem Rohrreaktor bei den bereits erwähnten Verweilzeiten von ≦ 1 s kontinuierlich durchgeführt; die dabei entstehenden Pyrolysegase werden sofort nach Verlassen der Hochtemperaturzone einem Quench in einer Flüssigkeit unterworfen. Als Kühlmedium haben sich inerte Flüssigkeiten als günstig erwiesen, die bevorzugt die Blausäure aus dem Acetylen-Blausäure-Gemisch absorbieren; geeignet ist beispielsweise eine Mischung aus wäßriger NaOH-Lösung und Dimethylformamid.

Das Abschrecken kann auch gemäß üblicher Verfahren, wie es etwa für das Öl-Quench-Acetylen-Verfahren (Ullmann's, Encyclopedia of Industrial Chemistry, 5. Ed.- 1985, Vol. A1, S. 107ff) und das Andrussow- oder das BMA-Verfahren zur Blausäure-Herstellung (Ullmann's Encyclopedia of Industrial Chemistry, 5. Ed.- 1987, Vol. A8, S. 161ff) beschrieben ist, erfolgen.

Ein Vorteil des erfindungsgemäßen Verfahrens gegenüber der Acetylen-Herstellung aus Methan bzw. Erdgas liegt darin, daß bei der Pyrolyse von Acrylnitril unter den beschriebenen Bedingungen praktisch kein Ruß gebildet wird.

### Beispiel

Ein aufrecht stehendes Reaktionsrohr (Durchmesser 4 cm, Länge 25 cm) wurde mit Keramikringen (Durchmesser 4 mm, Länge 4 mm) befüllt. Die Reaktionszone (Länge 2 cm, Durchmesser 4 cm) wurde mit einem glühenden Metallstreifen auf eine Temperatur von ca. 1000 °C beheizt. Mittels einer Pumpe wurde 15,7 ml/h (12,65 g/h) Acrylnitril in eine Verdampferzone eingebracht und dort mit 158 l/h Trägergas (Stickstoff) vermischt, wodurch ein Gasgemisch mit einem Acrylnitril-Anteil von 3,27 Vol.% resultierte. Dieses Gasgemisch wurde über die Reaktionszone geleitet, wobei die Verweilzeit in der Reaktionszone 0,1 s betrug.

Direkt hinter der Reaktionszone wurden die heißen Gase mit einem kalten Natronlauge/DMF-Gemisch abgeschreckt. Dabei wurde die Blausäure absorbiert. Das verbleibende Gas wurde anschließend getrocknet und durch Gassammelröhren geleitet. Die durch Gasanalyse bestimmte Acetylenmenge betrug 1,12 Vol.% entsprechend einer Acetylenausbeute von 34,2 %. Die nach einer Versuchsdauer von 34 Min. titrimetrisch bestimmte Blausäuremenge betrug 1,95 g, was einer Ausbeute von 53,4 % entsprach.

## Patentansprüche

1. Verfahren zur Herstellung von Acetylen und Blausäure, bei dem Acrylnitril einer Pyrolyse in einem Reaktor unterworfen wird und die bei der Pyrolyse gebildeten gasförmigen Reaktionsprodukte innerhalb von ≤ 1 s nach dem Verlassen der Pyrolysezone auf Temperaturen kleiner 100°C abgeschreckt werden.

2. Verfahren nach Anspruch 1, wobei die Pyrolyse des Acrylnitrils, gegebenenfalls unter Zusatz eines Inertgases, bei Temperaturen zwischen 900 und 1100°C und Verweilzeiten kleiner als 1 s durchgeführt wird.

3. Verfahren nach Anspruch 1 oder 2, wobei die Reaktionsprodukte in einer flüssigen Phase, bevorzugt mittels einer inerten Flüssigkeit abgeschreckt werden.

4. Verfahren nach einem der Ansprüche 1 bis 3, wobei die Pyrolyse kontinuierlich in einem rohrförmigen Reaktor durchgeführt wird.

## Claims

1. A process for preparing acetylene and hydrocyanic acid, which comprises pyrolyzing acrylonitrile in a reactor and quenching the gaseous reaction products of the pyrolysis down to less than 100°C within ≤ 1 s of leaving the pyrolysis zone.

2. A process as claimed in claim 1, wherein the pyrolyzing of the acrylonitrile is effected at 900 - 1100°C with a residence time of less than 1 s in the presence or absence of an inert gas.

3. A process as claimed in claim 1 or 2, wherein the reaction products are quenched in a liquid phase, preferably by means of an inert liquid.

4. A process as claimed in any of claims 1 to 3, wherein the pyrolyzing is effected continuously in a tubular reactor.

## Revendications

1. Procédé de production de l'acétylène et de l'acide cyanhydrique, dans lequel l'acrylonitrile est soumis à une pyrolyse dans un réacteur et les produits gazeux formés pendant la pyrolyse sont trempés en l'espace de ≤ 1 s après avoir quitté la zone de pyrolyse, à des températures inférieures à 100°C.

2. Procédé selon la revendication 1, dans lequel la pyrolyse de l'acrylonitrile, éventuellement avec ajout d'un gaz inerte, est réalisée à des températures comprises entre 900 et 1100°C et avec des durées de séjour inférieures à 1 s.

3. Procédé selon la revendication 1 ou 2, dans lequel les produits de réaction sont trempés dans une phase liquide, de préférence au moyen d'un liquide inerte.

4. Procédé selon l'une quelconque des revendications 1 à 3, dans lequel la pyrolyse est réalisée en continu dans un réacteur tubulaire.
